# FASCICULE DE BREVET EUROPEEN

(11) **EP 0 538 420 B1**
(45) Date de publication et mention de la délivrance du brevet: **02.11.1995**
(21) Numéro de dépôt: 92906536.5
(22) Date de dépôt: 19.02.1992
(51) Int. Cl.: A61F 2/14, A61L 27/00

(54) **SUPPORT DE MESOPROTHESE**
MESOPROTHESEUNTERLAGE
SUPPORT FOR A PARTIALLY EXTRACORPOREAL PROSTHESIS

(30) Priorité: 10.04.1991 FR 9104333
(43) Date de publication de la demande: 28.04.1993
(73) Titulaire: F.C.I. FRANCE CHIRURGIE INSTRUMENTATION, F-92130 Issy-Les Moulineaux (FR)
(72) Inventeur: LEGEAIS, Jean-Marc, F-94160 Saint-Mandé (FR)
(74) Mandataire: Viard, Jean
(86) Numéro de dépôt international: FR9200159
(87) Numéro de publication internationale: WO9218065

(56) Documents cités:
- EP-A- 0 369 034
- WO-A-89/00032
- FR-A- 2 608 041
- FR-A- 2 649 605
- US-A- 2 714 721
- US-A- 4 799 931

## Description

La présente invention a pour objet un support de mésoprothèse, c'est à dire d'une prothèse dont une partie se trouve à l'extérieur de l'organisme et la seconde à l'intérieur de celui-ci. Elle concerne en particulier, mais non exclusivement, les prothèses de cornée ou kératoprothèses.

Les kératoprothèses restent l'ultime recours des cécités cornéennes bilatérales lorsque les homogreffes sont inefficaces. Mais, étant donné la tendance de l'organisme à rejeter où à inclure tout corps étranger, la pose de telles prothèses entraîne de nombreuses complications qui sont notamment : l'hypertension oculaire, le décollement rétinien, les nécroses conjonctivales, les ulcérations sclérales et les expulsions prothétiques dont le taux est actuellement d'environ 50% à cinq ans.

Ces complications proviennent essentiellement de la liaison globe oculaire-prothèse. Afin d'améliorer la qualité de cette liaison ou haptique, il a déjà été proposé d'utiliser un support poreux colonisable par les cellules de manière à ce qu'un tissu cellulaire se reconstitue dans la zone de liaison. L'emploi de polytétrafluoroéthylène expansé (P.T.F.E.E. ou E.P.T.F.E.) est décrit dans le JOURNAL FRANCAIS D'OPHTALMOLOGIE, (1987, 10,6/7, pages 425 à 433) sous le titre "Etude d'un support en polytétrafluoroéthylène expansé" par MM. LEGEAIS et RENARD. Le document FR-A-2 649 605 décrit un support de mésoprothèse selon les caractéristiques du préambule de la revendication 1.

D'autres composés chimiques colonisables ont également été proposés dans FR-A-2 608 041.

Dans la technique actuellement pratiquée, on découpe dans une feuille de PTFEE, dont l'épaisseur est de 0,4 mm, un patch d'environ 10 mm de diamètre dont le centre est percé de manière à recevoir la lentille. Le diamètre moyen des pores est de 20 microns et l'on observe une colonisation des pores par les cellules. Toutefois, le PTFEE reste opaque c'est à dire blanc et la liaison hydrophobe.

La présente invention a pour objet de pallier cet inconvénient , de permettre une colonisation totale de l'haptique et de rendre la structure colonisée hydrophile.

Selon la présente invention, le support pour kératoprothèse constitué de P.T.F.E.E. est caractérisé en ce que les pores ou canaux de celui-ci sont parallèles et orientés perpendiculairement à la surface du support.

Selon une autre caractéristique de l'invention, le support a une épaisseur sensiblement égale à 0,2 millimètre. Le phénomène constaté de transparence disparaît du produit colonisé à partir d'une épaisseur d'environ 0,3 mm.

Enfin, le diamètre des pores ou canaux est compris entre 20 et 150 microns et, par exemple, de l'ordre de 50 microns.

Dans ces conditions, des coupes histologiques montrent une bonne colonisation et la présence de fibroblastes et de protéines à l'intérieur des canaux.

D'une façon surprenante, le support commence à devenir translucide après le dixième jour d'implantation et complètement transparent vers le vingtième, vingt cinquième jour ce qui constitue un avantage notable sur le plan esthétique.

De plus, le support qui est physiquement hydrophobe devient, après colonisation, hydrophile ce qui permet son irrigation. Tout se passe comme si le support proprement dit avait acquis de nouvelles propriétés physico-chimiques à savoir, modification de l'indice de réfraction et hydrophilie créant ainsi un organe hybride artificiel.

Il va de soi que de nombreuses variantes peuvent être introduites, notamment par substitution de moyens techniquement équivalents sans pour autant sortir du cadre de l'invention.

## Revendications

1. Support de mésoprothèse et, en particulier de kératoprothèse constitué à partir d'une couche de polytétrafluoroéthylène expanse (E.P.T.F.E.), caractérisé en ce que les canaux du E.P.T.F.E. sont parallèles entre eux et perpendiculaires à la surface.

2. Support selon la revendication 1, caractérisé en ce que son épaisseur est sensiblement égale à 0,2 millimètre.

3. Support selon l'une des revendications 1 ou 2, caractérisé en ce que le diamètre moyen des canaux est compris entre 20 et 150 microns.

## Claims

1. Support for a mesoprothesis and in particular for a keratoprosthesis, the support being made from a layer of expanded polytetrafluoroethylene (E.P.T.F.E.) characterised in that the channels of the E.P.T.F.E. are mutually parallel and perpendicular to the surface.

2. Support according to claim 1 characterized in that its thickness is substantially equal to 0,2 millimeters.

3. Support according to claim 1 or 2, characterized in that the mean diameter of the channels lies in the range of 20 to 150 microns.

## Patentansprüche

1. Mesoprothesenträger, insbesondere für eine Keratoprothese, der aus einer Schicht aus expandiertem Polytetrafluorethylen (EPTFE) besteht, dadurch gekennzeichnet, daß die Kanäle des EPTFE unter sich parallel und rechtwinklig zur Oberfläche sind.

2. Träger nach Anspruch 1, dadurch gekennzeichnet, daß seine Dicke im wesentlichen 0,2 mm beträgt.

3. Träger nach einem der Ansprüche 1 oder 2, dadurch gekennzeichnet, daß der mittlere Durchmesser der Kanäle zwischen 20 und 150 Mikron liegt.
